# EUROPEAN PATENT APPLICATION

(11) **EP 4 012 392 A1**
(43) Date of publication of application: **15.06.2022**
(21) Application number: 20213704.8
(22) Date of filing: 14.12.2020
(51) Int. Cl.: G01N 27/02, G01N 33/18

(54) **IDENTIFICATION OF COMPONENTS IN A FLUID FLOW USING ELECTRO IMPEDANCE SPECTROSCOPY**

(71) Applicant: Hypersoniq B.V., 2909 LA Capelle aan den IJssel (NL)
(72) Inventor: Kleczewski, Lazlo, 2909 LA Capelle aan den IJssel (NL)
(74) Representative: Kouzelis, Dimitrios

(57) **Abstract**

Method and apparatus for identification of one or more components in liquid and/or gaseous flows using Electro Impedance Spectrography, starting with low frequencies, and including later higher frequencies, such as infrared, ultraviolet, Xray etc. Preferably, a sweep over the different frequency bands takes place. Prior knowledge of the response of certain compounds is used where the compounds are known to a certain extent and the concentrations have to be monitored.

The apparatus may comprise more than two, preferably 3-12 modules for different frequency bands, ranging from 0.1Hz to 10GHz.

## Description

The present disclosure is related to a method and apparatus for identification using Electro Impedance Spectrography starting with low frequencies, such as to measure one or more component in liquid and/or gaseous flows. At a later stage the frequencies can also include the higher ranges, e.g. infrared, ultraviolet, Xray etc.

For various situations there is the need to identify the composition of unknown substances, varying from chemical process control to forensics. Various methods are in existence that can be used to identify compounds or substances, many of them mainly suitable for laboratory situations. Many sensors can only detect small number of compounds.

Electro Impedance Spectrography (EIS) can be used over a very wide detection range, currently in laboratory environments. Due to the ambiguity of the data obtained by this method, it is only suitable for detecting changes in concentrations of known particles, or for comparison to known references.

The present patent disclosure provides a method of measuring the concentration and/or constituents of a sample by electro impedance spectroscopy (EIS) in two or more frequency bands, wherein the measurements from the first frequency band are combined with measurements in the second frequency band such that the concentration of a certain constituent is established in real time (or near real time).

Preferably in the method a sweep over the different frequency bands takes place.

Preferably a rinsing takes place after each measurement.

Preferably also a priori knowledge is used in applications wherein the compounds are known to a certain extent and the concentrations have to be monitored.

Preferably the a priori knowledge is related to measurements of certain materials at earlier times; such information can be stored in memory

In a set-up wherein the constituents are known per se and are dissolved in a known solvent, such as water, and the concentrations are preferably measured around a peak and/or valley marking point in the Bode plot of at least one frequency band.

Typically the real part (i.e. the resistivity) of the Bode plot shows a peak in a range where certain solutions with constituents therein show a space charge polarization, such as in a frequency range at the lower frequencies, such as 0.1-100 Hz. The frequency are therefore preferably sufficiently distanced from each other, so that also other phenomena at very different frequencies are observed in the Bode plot.

At much higher frequencies such as in the range of 0.5-2 GHz in many instances a valley can be detected in the real part of the Bode plot due to Ionic relaxation and dipolar relaxation of compounds.

By combining different parts of the spectrum an unknown constituent in a known solvent can be detected unambiguously. Also parts showing changes of the complex part (i.e. capacitance) of the Bode plot can be used

Preferably the frequency bands comprise frequencies from 0.1 Hz- 30 GHz, preferably 10-100 kHz, 100 kHz-1 MHz, and/or 1 Mhz-1 GHz.

The present patent disclosure also provides an apparatus, comprising:
- a first module configured to provide voltage and/or electrical currents in a first frequency band and to measure the impedance of the sample in the first frequency band;
- a second module configured to provide voltage and/or electrical currents in a second frequency band different from the first band and to measure impedance in the second band.

Preferably the Apparatus comprises more than three, preferably 3-12 modules for different frequency bands, ranging from 0.1Hz-10GHz.

Using this apparatus, the measurements in different frequency bands can be executed (almost) simultaneously making (near) real time applications in monitoring and control feasible.

Preferably the apparatus comprises a housing, wherein the modules are arranged, as well as a system controller, data processor, power module, and/or one or more environmental sensors; the temperature in the housing preferably sufficiently controlled for reproducible measurements. The apparatus can be placed in the flow of waste water of an industrial or harbor site, or in a bypass of the main flow.

In this way such apparatus may also become mobile, which will be very useful for certain applications.

Preferably the apparatus is provided with a heating/cooling unit connected to a supply unit for providing cooling/heating fluid, and more preferably each module comprises a board provided with a temperature sensor connected to a secondary heating/cooling element for finely controlling the temperature of the EIS module.

The apparatus can be provided with AI(Artificial Intelligence) by using old measurements from data storage for learning purposes, or also using deep learning.

In this preferred embodiment the measurements will be independent of environmental conditions (temperature, vibrations, light etc.) as much as possible.

Further advantages, features and details of the present patentable subject matter will become apparent from the following description with reference to a drawing, in which show:
Fig. 1 a block diagram of a preferred embodiment of a sensor system according to the present invention;
Fig. 2 a block diagram of a preferred sensor block of the embodiment of fig. 1;
Fig. 3 a Bode Plot of measurements obtained by the preferred embodiment of fig.1;
Fig. 4 a Bode Plot of the impedance of characteristic point;
Fig. 5 and 6 resp. Bode plots of two solutions at different temperatures;
Fig. 7 a plot of a solution with an alloy dissolved in water; and
Fig. 8 a scheme explaining the way to identify an unknown material using the preferred embodiment of fig. 1 using 13 measurements.

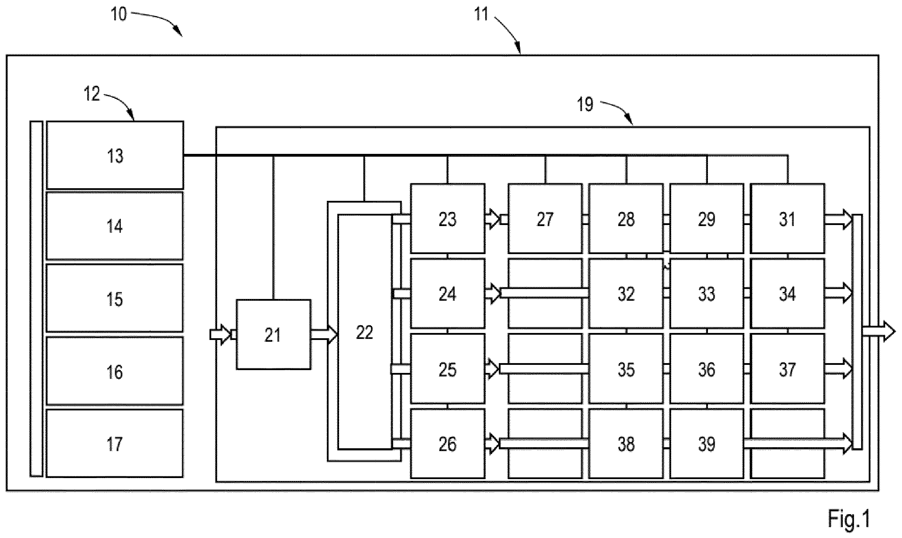

A preferred embodiment of a system 10 (fig. 1) comprises a housing 11, in which a main board 12 is arranged on which a system controller 13, a data processor 14, a communication unit 16, power unit and environmental sensors 18 are mounted.

In the housing 11 also a manifold on the backplate 19 is provided. Cooling/heating fluid is supplied by a pump 21 through a manifold element 22 to valve elements 23, 24, 25 and 26, resp. On the back plate 19 there are arranged twelve EIS measuring units 23-39 each configured to execute measurements in different frequency ranges, viz. unit 23 from 0.1-1 Hz, unit 28 from 1-10 Hz, unit 29 10-100 Hz, unit 31 from 0.1-1 kHz, unit 32 from 1-10 kHz, unit from 10-100 kHz, unit 34 from 0.1 MHz t0 1MHz, unit 35 from 1-10 MHz, unit 36 from 10-100 MHz, unit 37 from 0.1-1 GHz, unit 38 from 1-10 GHz and unit 39 from 10-100 GHz. On the backplate four further spaces are available to mount further measuring modules.

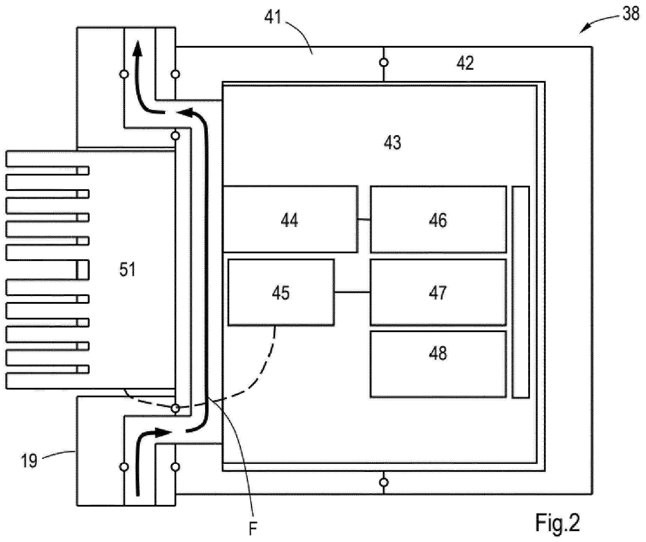

Each sensor module, e.g. 38 (fig. 2) comprises a housing 41, provided with a cover 42, preferably of metal (such as to form a Faraday shield against EM-waves), wherein a sensor board 43 is disposed. On the sensor board there are mounted an EIS sensor 44, temperature sensor 45, an EIS controller 46 and fine- tuning temperature controller 47 and a communication unit 48.

The cooling/heating fluid F flows from the backplate along the measuring module while heated/cooled by a finetuning heating/cooling element, which is electrically connected to the sensor 45 on the board and also to the controller 47 on the board.

Primary temperature control is executed by system controller 13 which is electrically connected to all measuring modules 23-39, to the primary heating/cooling element, as well as to the supply pump. As will be understood the temperature of the measuring modules has to be kept constant as much as possible during the measurement process. For that purpose usually additional cooling by an element such as 51 will be necessary for the module being active at a certain moment in time.

After each measurement, the system is preferably reset. A probe can be reset by rinsing with water with or without a chemical cleaning agent.

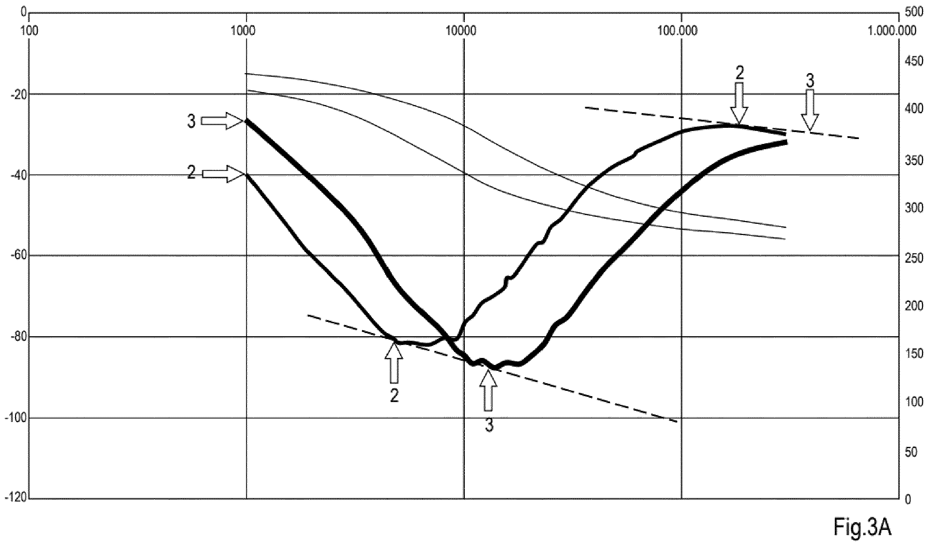

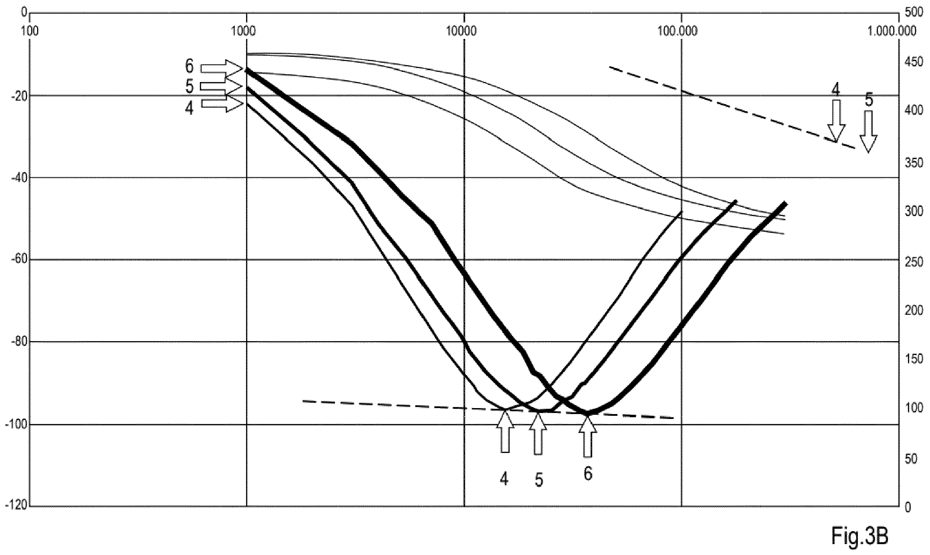

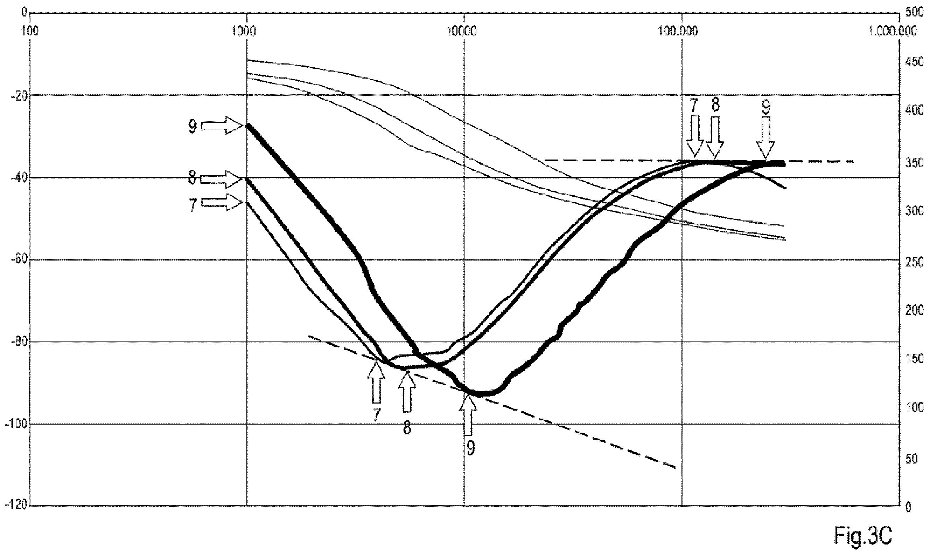

With the sensor system of fig. 2 a number a measurements were made in a frequency range of 1 kHz-300kHz; the results are shown in fig 3. Three salts were dissolved in three different concentrations in demineralized water:
Sample 1 ZnSO₄ (500ppm in H₂O) - not shown
Sample 2 ZnSO₄ (1000ppm in H₂O)
Sample 3 ZnSO₄ (2000ppm in H₂O)
Sample 4 FeNH₄(SO)₂ (500ppm in H₂O)
Sample 5 FeNH₄(SO)₂ (1000ppm in H₂O)
Sample 6 FeNH₄(SO)₂ (2000ppm in H₂O)
Sample 7 Pb(NO₃)₂ (500ppm in H₂O)
Sample 8 Pb(NO₃)₂ (1000ppm in H₂O)
Sample 9 Pb(NO₃)₂ (2000ppm in H₂O)

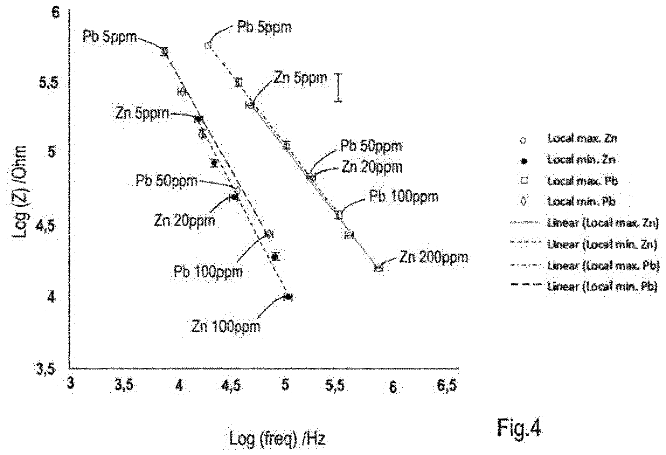

Further measurements were made for solutions of Pb(NO₃)₂ (Pb) and ZnSO₄ (Zn) (see Bode plot of fig. 4). The values of a local minimum and maximum for different concentration levels 5, 10, 20, 50 and 100 ppm resp. are on a straight line approximately and can be well distinguished from each other for the different concentrations (although Pb 50 ppm and Zn 20 ppm are somewhat close. Fig. 4 also shows the error margins, as the measurement were repeatedly executed.

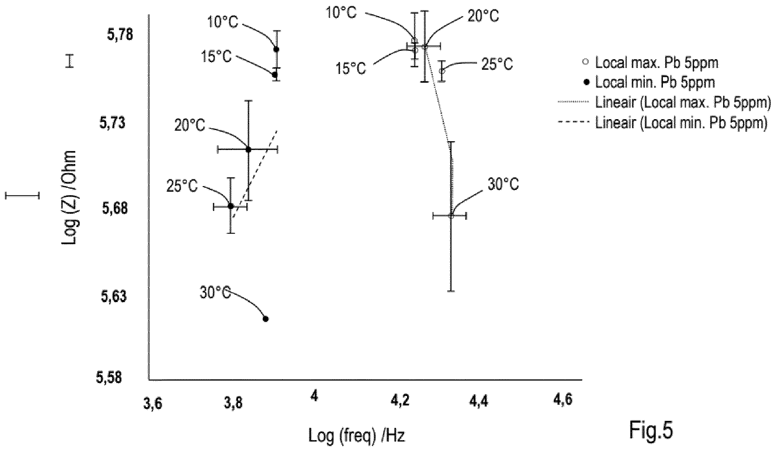

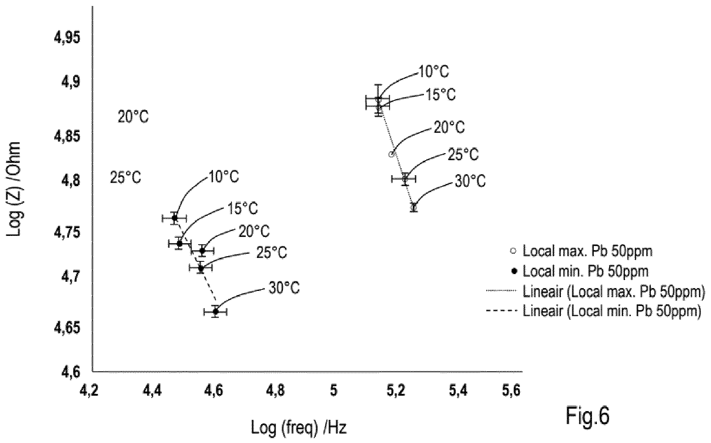

In the Bode plot of Fig. 5 and 6 the values Pb 5 and 50 ppm are shown for different temperatures. A deviation of 5 degrees Celsius will lead to less than 1% error margin.

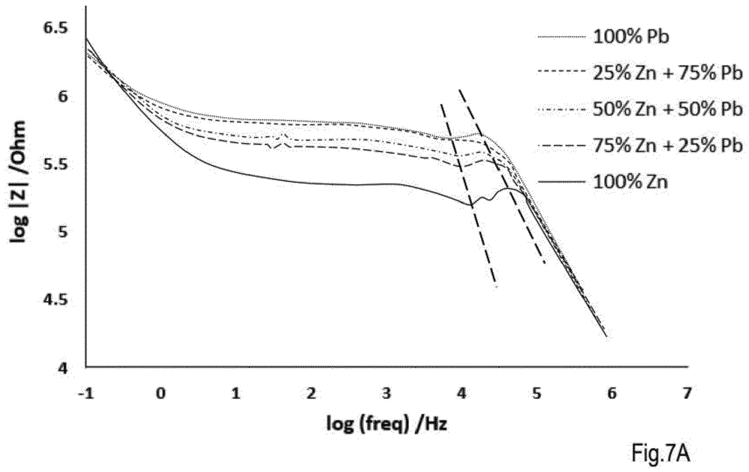

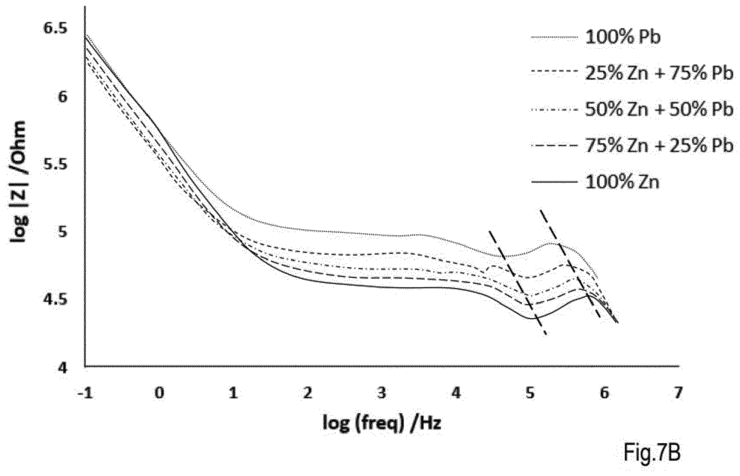

Fig. 7 shows that also mixed solutions with Pb and Zn are distinguishable for the different mixing ratios (at 20 degrees Celsius).

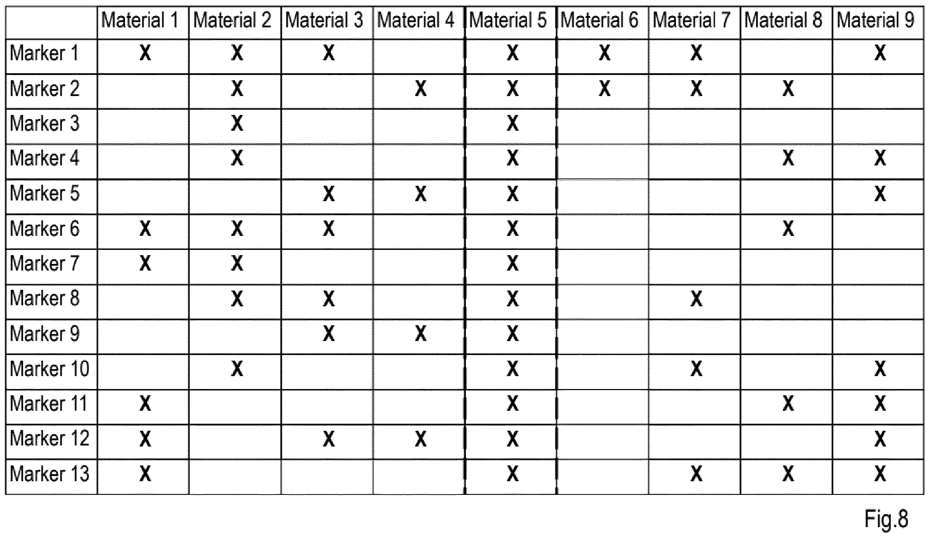

In the apparatus according to the description for instance thirteen marker frequencies could be chosen, markers 1-13 (fig. 8). By these thirteen marker frequencies a solution among nine materials 1-9 can be unambiguously identified in the right concentrations, i.d. material 5 to which all thirteen measurements correspond.

Data ambiguity is avoided in this way, so that a device is provide that autonomously can identifies the composition of unknown substances at real-time or near real-time (within a one or a few seconds).

Using the EIS technique, a few molecules with a large dipole effect can have the same result as a large number of molecules with a small dipole effect. Similar effects are present at other mechanisms EIS can detect, like the ion-relaxation, Space Charge Polarization or conductive regions.

Since the molecules of the substance to be measured affect the different EIS mechanisms in a different way, e.g. a large heavy molecule, with a small dipole, will have a larger effect on the Space Charge Polarization, with a smaller effect on the dipole relaxation. While a light molecule with a large dipole, will show the opposite behavior. Whereby each EIS region will show similar mechanism. This difference makes it possible to positively recognize the substances even if at one or more of the regions the measured results are similar.

Considering a priori knowledge of how individual substances or compounds behave in the different EIS regions, and a priori knowledge how combinations of compounds influence the measurements, by comparing these with measurements of an unknown substance, it becomes possible to deduct which substance and compounds fit the measurement and thus what the composition of the unknown substance must be.

This a priori knowledge could for example be how each of certain points in the Bode plot change in relation to substance composition, or concentration.

Further analysis of the data, like for example by looking at the real and imaginary signal parts, may yield additional points, or e.g. by using the complete data set as a fingerprint.

Once the substance is and its compounds are known, using a similar method, the concentration of each compound can be deducted by comparing the measured results to prior knowledge of the EIS behavior with different concentrations.

Further data analysis has shown that each compound can be detected independently, as the spectra of different constituents are superposed on each other.

By combining an EIS sensor with an algorithm for the substance identification, it is possible to autonomously identify materials in a real-time setting and allow immediate acting upon this identification.

As expected from literature samples 1-3 show measured minimum points on an (approximate straight line, as do samples 4-6 and samples 7-9. The measurements correspond with the theory and were also confirmed with an apparatus with limited frequency capabilities; in this respect it is herewith emphasized that the claims are not limited by any theory.

Furthermore, the theory learns that the different molecules (big/small, heavy/light, small/large dipole effect) of different substances will have a on The Space Charge Polarisation and a different effect on the dipole relaxation. Therefore, the different EIS regions can each make a different positive assessment of the molecules present.

It is the expectation that the present patent disclosure will make a major contribution in monitoring liquid and/or gaseous flows not only for water, e.g. in industrial sites but also in hydrocarbon applications as well as in other industries such as food where contaminants are undesirable, bridging the gap between laboratory and real life applications.

The present patent disclosure is not limited to the description, theory and embodiments above; the requested rights are determined by the following claims, within the scope of which many modifications are feasible.

## Claims

1. Method of measuring the concentration and/or constituents of a sample by electro impedance spectroscopy (EIS) in two or more frequency bands, wherein the measurements from the first frequency band are combined with measurements in the second frequency band such that the concentration of a certain constituent is established in real time (or near real time).

2. Method of claim 1, wherein also a priori knowledge is used in the combination.

3. Method of claim 2, wherein the a priori knowledge is related to measurements of certain materials at earlier times.

4. Method of claim 1,2 or 3, wherein the constituents known per se are dissolved in a known solvent, such as water, and the concentrations are measured around a peak and/or valley marking point in the Bode plot of at least one frequency band.

5. Method of claim 1,2 or 3, wherein an unknown constituent in a known solvent is detected unambiguously.

6. Method of any of claims 1-5, wherein the frequency bands comprise frequencies from 0.1 Hz- 30 GHz, preferably 10-100 kHz, 100 kHz-1 MHz, and/or 1 Mhz-1 GHz.

7. Method of any of claims 1-6, wherein the frequency is swept over the different frequencies.

8. Method of any of claims 1-7, wherein rinsing takes place after each measurement.

9. Apparatus, comprising:
- a first module configured to provide voltage and/or electrical currents in a first frequency band and to measure the impedance of the sample in the first frequency band;
- a second module configured to provide voltage and/or electrical currents in a second frequency band different from the first band and to measure impedance in the second band.

10. Apparatus according to claim 9, comprising 12 modules for different frequency bands, ranging from 0.1Hz to 10GHz.

11. Apparatus according to claim 9 or 10, comprising a housing, in which the modules are arranged, as well as a system controller, a data processor, a power module, and/or one or more environmental sensors.

12. Apparatus according to claim 9,10 or 11, provided with a heating/cooling unit connected to a supply unit for providing cooling/heating fluid.

13. Apparatus according to any of claims 9-12, wherein each module comprises a board provided with a temperature sensor connected to a secondary heating/cooling element for finely tuning the temperature control of the EIS module.

14. Apparatus according to any of claims 9-13, provided with memory for storing data from old measurements, either locally or, preferably, remotely in a network with on-line access.

15. Apparatus according to any of claims 9-14, provided with computer power for AI, or deep learning.
